# EUROPÄISCHE PATENTSCHRIFT

(11) **EP 0 755 912 B1**
(45) Veröffentlichungstag und Bekanntmachung des Hinweises auf die Patenterteilung: **08.09.1999**
(21) Anmeldenummer: 96109678.1
(22) Anmeldetag: 17.06.1996
(51) Int. Cl.: C07C 45/54, C07C 45/67, C07C 49/395

(54) **Verfahren zur Herstellung von 2-substituierten Cyclopentanonen**
Process for the preparation of 2-substituted cyclopentanones
Procédé de préparation de cyclopentanones 2-substituées

(30) Priorität: 28.06.1995 DE 19523448
(43) Veröffentlichungstag der Anmeldung: 29.01.1997
(73) Patentinhaber: BAYER AG, 51368 Leverkusen (DE)
(72) Erfinder: Müller, Nikolaus, Dr., 40789 Monheim (DE); Essert, Thomas, Dr., 51491 Overath (DE)

(56) Entgegenhaltungen:
- FR-A- 2 341 591
- SYNTHESIS, Nr. 12, Dezember 1983, Seiten 996-997, XP000616229 HENERSON, D. ET AL.: "A Quick and Efficient Route to 2-Substituted Cyclopentanones and cyclohexanones"
- POND, D.M. & CARGILL, R.L.: "Alkylation of 2-Carbethoxycyclopentanone in Dimethyl Sulfoxide", JOURNAL OF ORGANIC CHEMISTRY, , , Band 32, Nr. , Seiten 4064 - 4065

## Beschreibung

Die vorliegende Erfindung betrifft ein Verfahren zur Herstellung von 2-substituierten Cyclopentanonen, insbesondere von 2-Methylcyclopentanon aus Adipinsäuredialkylestern.

Es ist bereits bekannt, 2-substituierte Cyclopentanone durch Alkylierung von Cyclopentanon-2-carbonsäureestern und anschließende Verseifung und Decarboxylierung herzustellen. Dieses Verfahren ist mit einigen Nachteilen behaftet. Ausgehend von Adipinsäuredialkylestern ist das Verfahren dreistufig und ergibt selbst bei den besten in der Literatur beschriebenen Verfahren nur Ausbeuten von ca. 40 bis 45 % der Theorie. So führt die Cyclisierung beim Ethylester zu 70 bis 78 % isolierter Ausbeute, beim Methylester zu 68 bis 74 % isolierter Ausbeute (siehe J. prakt. Chem. 4. Reihe, Band 9, Seite 43 (1959)). Die anschließende Alkylierung z.B. mit Methylbromid zum 2-Methylcyclopentanon-2-carbonsäuremethylester gelingt in ca. 70 % Ausbeute der Theorie, die nachfolgende Verseifung und Decarboxylierung in ca. 80 % Ausbeute der Theorie, so daß sich eine Ausbeute von maximal 42 % der Theorie, bezogen auf eingesetzten Adipinsäuredialkylester ergibt (siehe Chem. Ber 80, 202 (1974)).

Grund für die niedrige Ausbeute ist die Reversibilität der Cyclisierung im ersten Schritt und die aus den gleichen Gründen stattfindende Spaltung durch die Hilfsbase Alkoholat bei der Bildung des Carbanions im Alkylierungsschritt. Durch die reversible Spaltung entsteht α-Alkyladipinsäureester, der seinerseits zum 5-Alkyl-cyclopentanon-2-carbonsäureester cyclisiert (siehe J. Org. Chem. 29, 2782 (1964)).

Zu Ausbeuteminderungen kommt es weiterhin durch O-Alkylierungen des mesomeren Enolations, die in ihrem Ausmaß stark von dem Alkaligegenion, dem Lösungsmittel und der Natur des Alkylierungsmittels abhängt. So führen gerade die preiswerten, häufig eingesetzten Alkylchloride und die Alkylsulfate zu erhöhter Bildung von O-Alkylderivaten, die bei energischer saurer Verseifung und anschließender Decarboxylierung Cyclopentanon ergeben, so daß bei der Isolierung, die meist durch Destillation erfolgt, ein erhöhter Trennaufwand nötig wird. Deshalb werden bei der Alkylierungsreaktion meist die teuren, arbeitshygienisch aufwendigen Alkylbromide und Alkyliodide eingesetzt.

Nachteilig bei diesen Verfahren wirkt sich auch die merkliche Wasserlöslichkeit der Endprodukte aus, die bei 2-Methylcyclopentanon besonders ausgeprägt ist. Diese führt dazu, daß die Produkte umständlich durch mehrfache Extraktion aus dem wäßrigen Reaktionsgemisch isoliert und die organischen Extrakte getrocknet und destilliert werden müssen. Die verbleibende wäßrige Phase ist mit organischen Stoffen belastet und bedarf einer besonderen Aufarbeitung und Entsorgung.

Für die Alkylierung des 2-Carboalkoxy-cyclopentanons mit dem entsprechenden Alkylhalogenid werden in der Literatur dipolare aprotische Lösungsmittel wie Dimethylsulfoxid (J. Org. Chem. 32, 4064 (1967)) und Aceton (SYNTHESIS 1973, 316) als vorteilhaft beschrieben, weil in den sonst üblichen unpolaren Lösungsmitteln wie Benzol oder Toluol höher molekulare Aggregate der Ionenpaare vorliegen, die beträchtlich weniger an freiem Enolation enthalten und damit deutlich schlechter reagieren (siehe J. Am. Chem. Soc. 82, 2895 (1960)).

Auch hier müssen Alkyliodide oder Alkylbromide eingesetzt werden, wobei der O-Alkylierungsanteil beim Bromid deutlich höher ist. Bei Einsatz von Alkylchloriden ist eine noch höhere O-Alkylierung zu erwarten, die das Verfahren noch unrentabler macht. Weiterhin ist eine spezifische Reaktion mit Kaliumsalzen des Cyclopentanon-carbonsäureesters beschrieben, was zur Folge hat, daß die Cyclisierung mit den wesentlich teureren Kaliumalkoholaten durchgeführt werden müßte, um bei der einstufigen Variante zu bleiben. Ansonsten macht der nötige Lösungsmittelwechsel das zweistufige Verfahren noch unwirtschaftlicher. Das Arbeiten in den dipolaren, aprotischen Lösungsmitteln schließt in der Regel folgende Aufarbeitungsvariante ein: Nach beendeter Reaktion wird der Alkylierungsansatz mit Wasser verdünnt und das Produkt durch Extraktion mit einem unpolaren Lösungsmittel wie Ether oder Kohlenwasserstoffen extrahiert. Wie schon oben diskutiert, erfordert die Polarität der Produkte ein mehrfaches Extrahieren, die Rückgewinnung der eingesetzten Lösungsmittel ist wegen ihrer vollständigen Mischbarkeit mit Wasser sehr aufwendig und es entstehen in der Regel stark organisch belastete Abwässer.

Die Verseifung von 2-Alkylcyclopentanon-2-carbonsäureester kann nur unter sauren Bedingungen erfolgen, da unter alkalischen Bedingungen die Nebenreaktion der Säurespaltung (= Ringöffnung) zu stark in den Vordergrund tritt. Bei der sauren Verseifung müssen starke Säuren eingesetzt werden, um zu einer brauchbaren Reaktionszeit zu kommen. So werden z.B. verdünnte Perchlorsäure (siehe Ber. 80, 202 (1974)) oder konzentrierte Salzsäure (siehe Bl. Soc. clim. Belg. 35, 315) eingesetzt, die wegen ihres höheren Preises und der stärker korrodierenden Eigenschaften weniger günstig sind als verdünnte Schwefelsäure.

Neben dem gängigsten, oben beschriebenen Verfahren, gibt es eine Reihe von weiteren Verfahren zur Herstellung von 2-Alkylcyclopentanonen, speziell 2-Methylcyclopentanon.

So beispielsweise die Cyclisierung von α-Methyladipinsäure zum Anhydrid und anschließend dessen thermische Zersetzung (siehe C.r. 144, 1357), die Calciumsalzdestillation dieser Säure und die direkte Alkylierung von Cyclopentanon, die allerdings zu einem kaum trennbaren Produktgemisch aus Mono-, Di- und Trimethylcyclopentanonen führt und nur mit starken Hilfsbasen, beispielsweise Natriumamid, gelingt.

Ausgehend von 1-Methylcyclopentenoxid kann man 2-Alkylcyclopentanone z.B. erhalten durch Behandlung mit Grignard-Verbindungen (siehe Soc. Chim. Belg. 37, 151), mit Perameisensäure/Schwefelsäure (siehe Am. Soc. 70, 4139 (1948)) oder durch Leiten des Dampfes über Aluminiumoxid bei 300°C.

Man kann auch 2-Chlor-1-methyl-cyclopentanol-(1) mit Grignard-Verbindungen umsetzen (siehe C.r. 209, 449 (1939)) oder mit Alkali behandeln (siehe Am. Soc. 96, 2783 (1934)).

Man kann auch verschiedene 2-Alkyliden-cyclopentanone reduzieren.

Schließlich beschreiben die FR-A 2 341 591 die Herstellung von 2-n-Butyl-2-carbethoxycyclopentanonen durch Umsetzung von Adipinsäureester mit Natrium in Gegenwart von Toluol zum 2-Natrium-2-carbethoxycyclopentanon, das getrocknet und dann mit Butylbromid in Gegenwart von Dimethylsulfoxid umgesetzt wird sowie Synthesis 1983, 996-997 die Umsetzung von Di-t-butyladipat mit Natriumhydrid, Neutralisierung des Reaktionsgemisches mit Essigsäure und eine komplizierte Aufarbeitung zur Herstellung von 2-t-Butoxycarbonylcyclopentanon und dessen Umsetzung, nach Isolierung, zu 2-substituierten Cyclopentanonen.

Alle diese Verfahren gehen von schwer zugänglichen Ausgangsmaterialien aus, benutzen technisch nur mit Aufwand zu handhabende Reagenzien und sind in vielen Fällen unselektiv und von geringer Ausbeute. Als selektive Methode bietet sich noch die Alkylierung des 1-Pyrrolidino-cyclopentens an (siehe J. Heterocycl. Chem. 28, 1293 (1991)), jedoch wird auch hier in der insgesamt 3-stufigen Synthese lediglich eine Ausbeute von 49 % der Theorie, bezogen auf Cyclopentanon, erhalten, das selbst wieder aus Adipinsäureestern hergestellt werden muß.

Es wurde nun ein Verfahren zur Herstellung von in 2-Stellung substituierten Cyclopentanonen der Formel 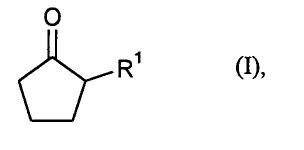 in der
- R¹: für geradkettiges oder verzweigtes C₁-C₆-Alkyl, C₃-C₇-Cycloalkyl oder gegebenenfalls durch Halogen substituiertes C₇-C₁₂-Aralkyl steht, gefunden,
das dadurch gekennzeichnet ist, daß man einen Adipinsäureester der Formel 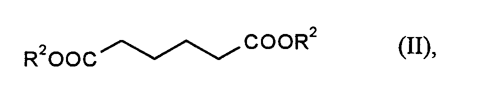 in der
die beiden R² gleich oder verschieden sind und jeweils für geradkettiges oder verzweigtes C₁-C₁₀-Alkyl, für gegebenenfalls mit bis zu 3 gleichen oder verschiedenen Substituenten aus der Gruppe der Halogene und der C₁-C₆-Alkyl-, C₁-C₆-Alkoxy- und Nitrogruppen substituiertes C₇-C₁₂-Aralkyl oder für gegebenenfalls mit bis zu 3 gleichen oder verschiedenen Substituenten aus der Gruppe der Halogene und der C₁-C₆-Alkyl-, C₁-C₆-Alkoxy- und Nitrogruppen substituiertes C₆-C₁₀-Aryl stehen,
in Gegenwart eines inerten Lösungsmittels mit einem Alkoholat der Formel (III) umsetzt

M(OR³)ₙ (III),

in der
- M: für ein Alkali- oder Erdalkalimetall,
- R³: für C₁-C₁₀-Alkyl, C₃-C₁₀-Cycloalkyl oder C₇-C₁₂-Aralkyl und
- n: je nach der Wertigkeit von M für 1 oder 2 stehen, so ein Salz des Cyclopentanon-2-carbonsäureesters der Formel (IV) erhält 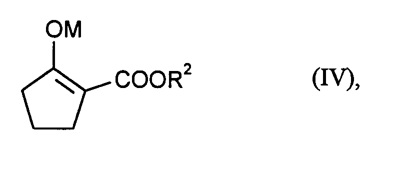
in der
- R²: die bei Formel (II) angegebene Bedeutung hat, und
- M: für ein Äquivalent eines Alkali- oder Erdalkalimetalls steht,
dieses ohne Isolierung mit einem Alkylierungsmittel der Formel (V) alkyliert,

R¹-X (V),

in der
- R¹: die bei Formel (I) angegebene Bedeutung hat und
- X: für Halogen, C₁-C₆-Alkylsulfonat oder gegebenenfalls mit bis zu zwei C₁-C₄-Alkylgruppen substituiertes C₆-C₁₀-Arylsulfonat steht,
so einen 2-Alkyl-cyclopentanon-2-carbonsäureester der Formel (VI) erhält 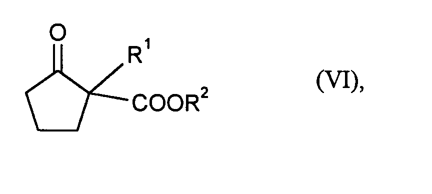 in der
- R²: die bei Formel (II) und
- R¹: die bei Formel (I) angegebene Bedeutung haben,
und diesen ohne Isolierung durch Behandeln mit einer Säure und Erhitzen verseift und decarboxyliert.

Halogen steht vorzugsweise für Chlor oder Brom, insbesondere für Chlor.

Von den Adipinsäureestern der Formel (II) sind solche bevorzugt, bei denen die beiden R²-Reste gleich sind und für geradkettiges oder verzweigtes C₁-C₆-Alkyl, gegebenenfalls durch bis zu zwei Substituenten aus der Gruppe der Halogenatome und der C₁-C₄-Alkylgruppen substituiertes Benzyl oder gegebenenfalls durch bis zu zwei Substituenten aus der Gruppe der C₁-C₄-Alkylgruppen und der Nitrogruppe substituiertes Phenyl stehen.

Besonders bevorzugt sind Adipinsäureester der Formel (II), bei denen die beiden R²-Reste gleich sind und für geradkettiges C₁-C₄-Alkyl stehen. Insbesondere bevorzugt sind Adipinsäuredimethylester und Adipinsäurediethylester.

Von den Alkoholaten der Formel (III) sind solche bevorzugt, bei denen M für Natrium, Kalium, Lithium, Calcium oder Magnesium und R³ für geradkettiges oder verzweigtes C₁-C₆-Alkyl, C₃-C₇-Cycloalkyl oder Benzyl stehen. Besonders bevorzugt sind Natrium-, Kalium-, Calcium- und Magnesiummethylat, -ethylat, -n-propylat, -i-propylat, -n-butylat, -i-butylat, -sek.-butylat und -t-butylat sowie Natrium- und Kaliumpentylat, -hexylat, -cyclopentylat, -cyclohexylat und - benzylat. Insbesondere bevorzugt sind Natrium- und Kaliummethylat, -ethylat, -i-propylat und -t-butylat.

Als inerte Lösungsmittel für die Umsetzung des Adipinsäureesters mit dem Alkoholat kommen beispielsweise aliphatische und aromatische Kohlenwasserstoffe mit Siedepunkten von mindestens 80°C (bei Normaldruck) in Frage. Beispielsweise seien genannt: Heptane, Octane, Nonane, Decane, Isododecane, Benzol, Toluol, Cumole, Xylole, Di- und Triisopropylbenzole und durch Halogen und/oder C₁-C₄-Alkoxy substituierte aromatische Kohlenwasserstoffe wie Chlorbenzol, Dichlorbenzole, Trichlorbenzole, Anisol, Phenetol und Phenylisopropylether. Es können auch beliebige Gemische dieser Lösungsmittel eingesetzt werden.

Den zuvor beschriebenen Lösungsmitteln kann man gegebenenfalls dipolare, aprotische Lösungsmittel zufügen, beispielsweise Dimethylsulfoxid, Tetramethylensulfon, Di-C₁-C₆-(cyclo)-alkylformamide, N-C₁-C₆-alkylpyrrolidone, -piperidone und/oder -caprolactame, Tetra-C₁-C₆-alkylharnstoffe und N,N'-Dimethylethylen- und N,N'-Dimethylpropylenharnstoff. Der Anteil dipolarer, aprotischer Lösungsmittel an insgesamt eingesetzten Lösungsmitteln kann z.B. 0 bis 50 Gew.-% betragen. Vorzugsweise beträgt dieser Anteil 0 bis 20 Gew.-%, ganz besonders bevorzugt ist kein dipolar aprotisches Lösungsmittel vorhanden oder beträgt sein Anteil 0,01 bis 10 Gew.-%.

Bezogen auf 1 Mol Adipinsäureester der Formel (II) kann man z.B. 500 bis 5000 ml Lösungsmittel einsetzen. Vorzugsweise liegt diese Menge bei 800 bis 3000 ml, insbesondere bei 1000 bis 2000 ml.

In die Umsetzung mit dem Adipinsäureester der Formel (II) kann man das Alkoholat der Formel (III) z.B. als trockenen, vorzugsweise feinpulvrigen Feststoff oder als Lösung in dem dem jeweiligen Alkoholat zugrundeliegenden Alkohol einsetzen. Vorzugsweise verfährt man so, daß man das Alkoholat in Lösung in dem dem jeweiligen Alkoholat zugrundeliegenden Alkohol einsetzt und den Alkohol abdestilliert bevor die Reaktion mit dem Adipinsäureester der Formel (II) in nennenswertem Umfang einsetzt. Auf diese Weise entsteht eine feinverteilte Suspension des Alkoholat im Reaktionsgemisch, was zu besonders guten Ergebnissen führt. So wird auch die Handhabung von staubbildenden und hygroskopischen festen Alkoholaten vermieden, die deswegen und wegen ihrer ätzenden Wirkung besondere arbeitshygienische Maßnahmen erfordern würden.

Bezogen auf 1 Mol Adipinsäureester der Formel (II) kann man beispielsweise 0,1 bis 10 Äquivalente eines Alkoholats der Formel (III) einsetzen. Dabei entspricht 1 Mol eines Alkoholats der Formel (III) mit M = einem Alkalimetall einem Äquivalent und 1 Mol eines Alkoholats der Formel (III) mit M = einem Erdalkalimetall zwei Äquivalenten. Vorzugsweise werden pro Mol Adipinsäureester 0,5 bis 2 Äquivalente Alkoholat, insbesondere pro Mol Adipinsäureester 0,95 bis 1,05 Äquivalente Alkoholat eingesetzt.

Die Umsetzung des Adipinsäureesters kann man z.B. bei Temperaturen im Bereich 80 bis 200°C durchführen. Bevorzugt sind Temperaturen im Bereich 100 bis 150°C. Besonders bevorzugt arbeitet man bei der Temperatur, bei der das jeweils eingesetzte inerte Lösungsmittel siedet. Es ist vorteilhaft, den bei der Umsetzung des Adipinsäureesters mit dem Alkoholat entstehenden Alkohol fortlaufend abzudestillieren.

Um eine gute Rührbarkeit während des gesamten Reaktionsablaufs zu gewährleisten, kann es gegebenenfalls erforderlich werden, während der Reaktion zusätzliches inertes Lösungsmittel und/oder dipolare, aprotische Lösungsmittel der oben beschriebenen Art zuzufügen.

Falls der bei der Umsetzung des Adipinsäureesters mit dem Alkoholat entstandene Alkohol nicht schon während der Umsetzung abdestilliert wurde, wird er nach Beendigung der Umsetzung abdestilliert. Das praktisch von Alkohol freie Reaktionsgemisch, das das Salz des gebildeten Cyclopentanon-2-carbonsäureesters der Formel (IV) enthält, wird nun der Umsetzung mit einem Alkylierungsmittel der Formel (V) zugeführt.

Bevorzugte Alkylierungsmittel der Formel (V) sind solche, bei denen R¹ für Methyl, Ethyl, n-Propyl, i-Propyl, Benzyl, 4-Chlorbenzyl oder 4-Fluorbenzyl und X für Chlor, Brom oder p-Tolylsulfonat stehen. Besonders bevorzugt steht R¹ für Methyl, Ethyl, i-Propyl oder 4-Chlorbenzyl und X für Chlor. Insbesondere wird Methylchlorid eingesetzt.

Die Alkylierung kann im gleichen Reaktionsgefäß durchgeführt werden, in welchem die Umsetzung des Adipinsäureesters mit dem Alkoholat erfolgte. Wenn man flüchtige Alkylierungsmittel der Formel (V) einsetzt, ist es vorteilhaft, unter Druck bzw. im geschlossenen Gefäß zu arbeiten. Man kann dann das Salz des Cyclopentanon-2-carbonsäureesters der Formel (IV) enthaltende Gemisch gegebenenfalls in ein für das Arbeiten unter Druck geeignetes Reaktionsgefäß überführen.

Bezogen auf 1 Mol ursprünglich eingesetzten Adipinsäureester der Formel (II) kann man z.B. 1 bis 20 Mol eines Alkylierungsmittels der Formel (V) einsetzen. Vorzugsweise liegt diese Menge bei 1 bis 10 Mol, insbesondere bei 1 bis 5 Mol.

Die Reaktionstemperatur für die Alkylierung kann beispielsweise im Bereich 0 bis 250°C liegen. Vorzugsweise liegt sie im Bereich 20 bis 200°C, insbesondere im Bereich 50 bis 160°C. Welche Temperatur für welches Alkylierungsmittel optimal ist, kann gewünschtenfalls durch routinemäßige Reihenversuche leicht ermittelt werden.

Es ist vorteilhaft nach der Alkylierung das oder die Lösungsmittel und evtl. vorhandenes überschüssiges Alkylierungsmittel aus dem Reaktionsgemisch zu entfernen, z.B. durch Destillation. Eine Isolierung des gebildeten 2-Alkyl-cyclopentanon-2-carbonsäureesters der Formel (VI) ist nicht notwendig.

Zur Behandlung des 2-Alkyl-cyclopentanon-2-carbonsäureesters der Formel (VI) mit einer Säure sind beispielsweise starke organische und anorganische Säuren geeignet. Bevorzugt sind Bromwasserstoffsäure, Schwefelsäure, Alkylsulfonsäuren und Arylsulfonsäuren. Besonders bevorzugt ist Schwefelsäure.

Die Säuren werden zweckmäßigerweise in Form wäßriger Lösungen eingesetzt, z.B. als 5 bis 99 gew.-%ige wäßrige Lösungen. Vorzugsweise liegt die Konzentration der Säuren zwischen 10 und 80 Gew.-%, insbesondere zwischen 20 und 50 Gew.-%.

Bezogen auf 1 Mol ursprünglich eingesetzten Adipinsäureester der Formel (II) kann man beispielsweise 1 bis 10, vorzugsweise 1 bis 5 Äquivalente Säure einsetzen.

Die Verseifung und Decarboxylierung kann z.B. bei Temperaturen im Bereich 50 bis 150°C durchgeführt werden. Vorzugsweise arbeitet man bei der Siedetemperatur des wäßrigen Reaktionsgemisches.

Aus dem Reaktionsgemisch kann das hergestellte, in 2-Stellung substituierte 2-Cyclopentanon der Formel (I) z.B. durch azeotrope Destillation isoliert werden.

Eine bevorzugte Ausführungsform des erfindungsgemäßen Verfahrens wird wie folgt durchgeführt:

Inertes Lösungsmittel und alkoholische Alkoholatlösung werden vorgelegt. Dann gibt man Adipinsäureester zu und destilliert während dessen Umsetzung mit dem Alkoholat den überschüssigen und den gebildeten Alkohol ab. Nach Beendigung dieser Reaktion wird Alkylierungsmittel zugefügt und nach beendeter Alkylierung das inerte Lösungsmittel abdestilliert. Der Rückstand wird mit starker wäßriger Säure versetzt und am Rückfluß gekocht. Abschließend wird 2-substituiertes Cyclopentanon durch azeotrope Destillation aus dem Reaktionsgemisch isoliert.

Das erfindungsgemäße Verfahren gestattet die Herstellung von 2-substituierten Cyclopentanonen der Formel (I) aus Adipinsäureestern der Formel (II) ohne Isolierung der Zwischenprodukte, ohne Trocknungsschritte, ohne mehrfache Abtrennung von Lösungsmitteln, ohne Lösungsmittelwechsel und ohne aufwendige Trennoperationen. Die Ausbeute liegt dabei sehr hoch, im allgemeinen bei über 80 % der Theorie. Das erfindungsgemäße Verfahren ist deshalb energiesparend und besonders wirtschaftlich.

2-substituierte Cyclopentanone der Formel (I) sind wertvolle Zwischenprodukte zur Herstellung von Schädlingsbekämpfungsmitteln, speziell Fungiziden (siehe z.B. EP-A 329 397, EP-A 378 953 und EP-A 537 909). Sie können auch als solche oder nach weiterer Umwandlung als Riech- oder Aromastoffe eingesetzt werden.

### Beispiele

### Beispiel 1

In einen 3 l-Vierhalskolben wurden 348,4 g Adipinsäuredimethylester, 381 ml 30 gew.-%ige Natriummethylatlösung in Methanol und 2884 ml Toluol vorgelegt und innerhalb von 2,5 Stunden Methanol abdestilliert. Danach wurde geheizt und bei einer Kopftemperatur von 100°C in 1,5 Stunden ein Gemisch aus Methanol und Toluol abdestilliert. Der Rückstand wurde in einen 3 l Edelstahl-Autoklaven überführt, 50 g Methylchlorid aufgedrückt und auf 140°C erhitzt. Nach 1 Stunde wurden weitere 50 g Methylchlorid, nach 2 Stunden noch einmal 52 g Methylchlorid aufgedrückt. Danach wurde weitere 5 Stunden bei 150°C nachgerührt.

Der Ansatz wurde abgekühlt und in eine 3 l-Vierhalskolbenrührapparatur mit Destillationsaufsatz überführt. Die Hauptmenge des Toluols wurde bei Normaldruck, Reste bei 33 mbar bis zu einer Sumpftemperatur von 90°C abdestilliert. Die zurückbleibende Suspension wurde mit 981 g 20 gew.-%iger Schwefelsäure versetzt und 7 Stunden am Rückfluß gekocht. Anschließend wurde das Produkt über einen Wasserabscheider azeotrop abdestilliert (Kopftemperatur bei Normaldruck: 92 bis 100°C). Es wurden 212,5 g eines wasserklaren Produktes mit einem Gehalt von 77,7 Gew.-% 2-Methylcyclopentanon und 22 Gew.-% Wasser erhalten. Die Ausbeute betrug 84,2 % bezogen auf eingesetzten Adipinsäuredimethylester.

Durch Extraktion und/oder Destillation kann gewünschtenfalls das Wasser aus dem erhaltenen 2-Methylcyclopentanon entfernt werden.

## Patentansprüche

1. Verfahren zur Herstellung von in 2-Stellung substituierten Cyclopentanonen der Formel 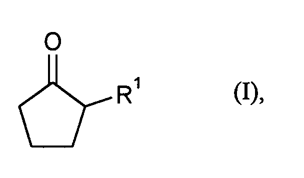 in der
R¹ für geradkettiges oder verzweigtes C₁-C₆-Alkyl, C₃-C₇-Cycloalkyl oder gegebenenfalls durch Halogen substituiertes C₇-C₁₂-Aralkyl steht,
dadurch gekennzeichnet, daß man einen Adipinsäureester der Formel 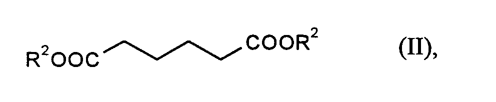 in der
die beiden R² gleich oder verschieden sind und jeweils für geradkettiges oder verzweigtes C₁-C₁₀-Alkyl, für gegebenenfalls mit bis zu 3 gleichen oder verschiedenen Substituenten aus der Gruppe der Halogene und der C₁-C₆-Alkyl-, C₁-C₆-Alkoxy- und Nitrogruppen substituiertes C₇-C₁₂-Aralkyl oder für gegebenenfalls mit bis zu 3 gleichen oder verschiedenen Substituenten aus der Gruppe der Halogene und der C₁-C₆-Alkyl-, C₁-C₆-Alkoxy- und Nitrogruppen substituiertes C₆-C₁₀-Aryl stehen,
in Gegenwart eines inerten Lösungsmittels mit einem Alkoholat der Formel (III) umsetzt
M(OR³)ₙ (III),
in der
M für ein Alkali- oder Erdalkalimetall,
R³ für C₁-C₁₀-Alkyl, C₃-C₁₀-Cycloalkyl oder C₇-C₁₂-Aralkyl und
n je nach der Wertigkeit von M für 1 oder 2 stehen,
so ein Salz des Cyclopentanon-2-carbonsäureesters der Formel (IV) erhält 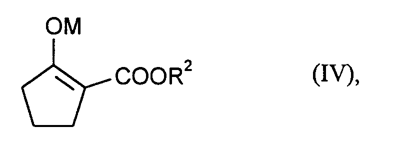 in der
R² die bei Formel (II) angegebene Bedeutung hat und
M für ein Äquivalent eines Alkali- oder Erdalkalimetalls steht,
dieses ohne Isolierung mit einem Alkylierungsmittel der Formel (V) alkyliert,
R¹-X (V),
in der
R¹ die bei Formel (I) angegebene Bedeutung hat und
X für Halogen, C₁-C₆-Alkylsulfonat oder gegebenenfalls mit bis zu zwei C₁-C₄-Alkylgruppen substituiertes C₆-C₁₀-Arylsulfonat steht,
so einen 2-Alkyl-cyclopentanon-2-carbonsäureester der Formel (VI) erhält 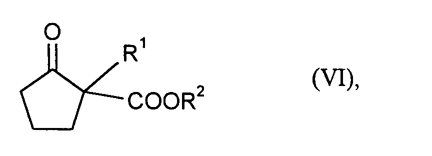 in der
R² die bei Formel (II) und
R¹ die bei Formel (I) angegebene Bedeutung haben,
und diesen ohne Isolierung durch Behandeln mit einer Säure und Erhitzen verseift und decarboxyliert.

2. Verfahren nach Anspruch 1, dadurch gekennzeichnet, daß bei Formel (II) beide R²-Reste gleich sind und für geradkettiges oder verzweigtes C₁-C₆-Alkyl, gegebenenfalls durch bis zu zwei Substituenten aus der Gruppe der Halogenatome und der C₁-C₄-Alkylgruppen substituiertes Benzyl oder gegebenenfalls durch bis zu zwei Substituenten aus der Gruppe der C₁-C₄-Alkylgruppen und der Nitrogruppe substituiertes Phenyl stehen, in Formel (III) M für Natrium, Kalium, Lithium, Calcium oder Magnesium und R³ für geradkettiges oder verzweigtes C₁-C₆-Alkyl, C₃-C₇-Cycloalkyl oder Benzyl stehen, als Lösungsmittel aliphatische oder aromatische Kohlenwasserstoffe mit Siedepunkten von mindestens 80°C (bei Normaldruck) oder durch Halogen und/oder C₁-C₄-Alkoxy substituierte aromatische Kohlenwasserstoffe oder Gemische dieser Lösungsmittel eingesetzt werden und das Alkoholat der Formel (III) als Lösung in den dem jeweiligen Alkoholat zugrundeliegenden Alkohol einsetzt und den Alkohol abdestilliert bevor die Reaktion mit dem Adipinsäureester der Formel (II) in nennenswertem Umfang einsetzt.

3. Verfahren nach Ansprüchen 1 und 2, dadurch gekennzeichnet, daß man dem Lösungsmittel zusätzlich dipolare aprotische Lösungsmittel hinzufügt.

4. Verfahren nach Ansprüchen 1 bis 3, dadurch gekennzeichnet, daß man pro Mol Adipinsäureester der Formel (II) 0,1 bis 10 Äquivalente eines Alkoholats der Formel (III) einsetzt und die Umsetzung bei Temperaturen im Bereich 80 bis 200°C durchführt.

5. Verfahren nach Ansprüchen 1 bis 4, dadurch gekennzeichnet, daß man als Alkylierungsmittel der Formel (V) solche einsetzt, bei denen R¹ für Methyl, Ethyl, n-Propyl, i-Propyl, Benzyl, 4-Chlorbenzyl oder 4-Fluorbenzyl und X für Chlor, Brom oder p-Tolylsulfonat stehen.

6. Verfahren nach Ansprüchen 1 bis 5, dadurch gekennzeichnet, daß man auf 1 Mol Adipinsäureester der Formel (II) 1 bis 20 Mol eines Alkylierungsmittels der Formel (V) einsetzt, die Alkylierung bei Temperaturen im Bereich 0 bis 250°C durchführt und nach der Alkylierung die Lösungsmittel und eventuell vorhandenes überschüssiges Alkylierungsmittel entfernt.

7. Verfahren nach Ansprüchen 1 bis 6, dadurch gekennzeichnet, daß man die Behandlung mit einer Säure mit Bromwasserstoffsäure, Schwefelsäure, Alkylsulfonsäuren oder Arylsulfonsäuren durchführt und die Säuren in Form wäßriger Lösungen einsetzt.

8. Verfahren nach Ansprüchen 1 bis 7, dadurch gekennzeichnet, daß man auf 1 Mol Adipinsäureester der Formel (II) 1 bis 10 Äquivalente Säure einsetzt.

9. Verfahren nach Ansprüchen 1 bis 8, dadurch gekennzeichnet, daß man das hergestellte, in 2-Stellung substituierte 2-Cyclopentanon der Formel (I) durch azeotrope Destillation isoliert.

## Claims

1. Process for the preparation of 2-substituted cyclopentanones of the formula 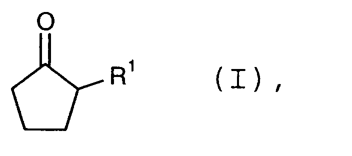 in which
R¹ represents straight-chain or branched C₁-C₆-alkyl, C₃-C₇-cycloalkyl or unsubstituted or halogen-substituted C₇-C₁₂-aralkyl,
characterized in that an adipic ester of the formula 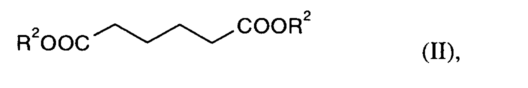 in which
the two R² are identical or different and each represent straight-chain or branched C₁-C₁₀-alkyl, C₇-C₁₂-aralkyl which may optionally be substituted by up to 3 identical or different substituents selected from the group consisting of the halogens and the C₁-C₆-alkyl, C₁-C₆-alkoxy and nitro groups, or C₆-C₁₀-aryl which may optionally be substituted by up to 3 identical or different substituents selected from the group consisting of the halogens and the C₁-C₆-alkyl, C₁-C₆-alkoxy and nitro groups,
is reacted in the presence of an inert solvent with an alkoxide of the formula (III)
M(OR³)ₙ (III),
in which
M represents an alkali metal or an alkaline earth metal,
R³ represents C₁-C₁₀-alkyl, C₃-C₁₀-cycloalkyl or C₇-C₁₂-aralkyl and
n, depending on the valency of M, represents 1 or 2,
a salt of the cyclopentanone-2-carboxylic ester of the formula (IV) is thus obtained 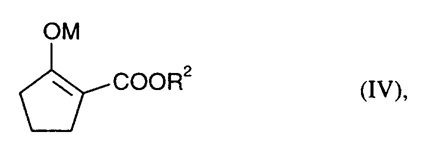 in which
R² has the meaning given under formula (II), and
M represents one equivalent of an alkali metal or alkaline earth metal,
this, without isolation, is alkylated with an alkylating agent of the formula (V),
R¹-X (V),
in which
R¹ has the meaning given under formula (I) and
X represents halogen, C₁-C₆-alkylsulphonate, or C₆-C₁₀-arylsulphonate which may optionally be substituted by up to two C₁-C₄-alkyl groups,
a 2-alkyl-cyclopentanone-2-carboxylic ester of the formula (VI) is thus obtained 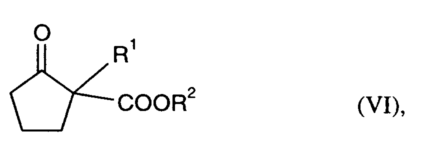 in which
R² has the meaning given under formula (II) and
R¹ has the meaning given under formula (I),
and this, without isolation, is hydrolysed and decarboxylated by treating it with an acid and heating.

2. Process according to Claim 1, characterized in that, in formula (II), the two R² radicals are identical and represent straight-chain or branched C₁-C₆-alkyl, benzyl which may optionally be substituted by up to two substituents selected from the group consisting of the halogen atoms and the C₁-C₄-alkyl groups, or phenyl which may optionally be substituted by up to two substituents selected from the group consisting of the C₁-C₄-alkyl groups and the nitro group, in formula (III), M represents sodium, potassium, lithium, calcium or magnesium and R³ represents straight-chain or branched C₁-C₆-alkyl, C₃-C₇-cycloalkyl or benzyl, as solvent, use is made of aliphatic or aromatic hydrocarbons having boiling points of at least 80°C (at atmospheric pressure) or aromatic hydrocarbons substituted by halogen and/or C₁-C₄-alkoxy, or mixtures of these solvents, and the alkoxide of the formula (III) is used as a solution in the alcohol underlying the respective alkoxide and the alcohol is distilled off before the reaction with the adipic ester of the formula (II) starts to a significant extent.

3. Process according to Claims 1 and 2, characterized in that dipolar aprotic solvents are additionally added to the solvent.

4. Process according to Claims 1 to 3, characterized in that, per mol of adipic ester of the formula (II), 0.1 to 10 equivalents of an alkoxide of the formula (III) are used and the reaction is carried out at temperatures in the range 80 to 200°C.

5. Process according to Claims 1 to 4, characterized in that, as alkylating agents of the formula (V), use is made of those in which R¹ represents methyl, ethyl, n-propyl, i-propyl, benzyl, 4-chlorobenzyl or 4-fluorobenzyl and X represents chlorine, bromine or p-tolylsulphonate.

6. Process according to Claims 1 to 5, characterized in that, per mol of adipic ester of the formula (II), 1 to 20 mol of an alkylating agent of the formula (V) are used, the alkylation is carried out at temperatures in the range 0 to 250°C and, after the alkylation, the solvent and any excess alkylating agent present are removed.

7. Process according to Claims 1 to 6, characterized in that the acid treatment is carried out with hydrobromic acid, sulphuric acid, alkylsulphonic acids or arylsulphonic acids and the acids are used in the form of aqueous solutions.

8. Process according to Claims 1 to 7, characterized in that, per mol of adipic ester of the formula (II), 1 to 10 equivalents of acid are used.

9. Process according to Claims 1 to 8, characterized in that the prepared 2-substituted 2-cyclopentanone of the formula (I) is isolated by azeotropic distillation.

## Revendications

1. Procédé pour la préparation de cyclopentanones substituées en position 2 et répondant à la formule 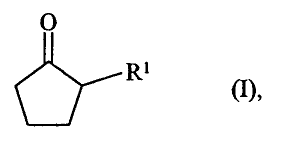 dans laquelle
R¹ représente un groupe alkyle à chaîne droite ou ramifiée en C₁-C₆, cycloalkyle en C₃-C₇ ou un groupe aralkyle en C₇-C₁₂ éventuellement substitué par des halogènes,
caractérisé en ce que l'on fait réagir un ester adipique de formule : 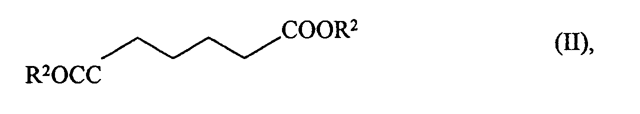 dans laquelle
les deux symboles R², ayant des significations identiques ou différentes, représentent chacun un groupe alkyle à chaîne droite ou ramifiée en C₁-C₁₀, un groupe aralkyle en C₇-C₁₂ portant éventuellement jusqu'à trois substituants identiques ou différents choisis parmi les halogènes et les groupes alkyles en C₁-C₆, alcoxy en C₁-C₆ et nitro, ou un groupe aryle en C₆-C₁₀ portant éventuellement jusqu'à trois substituants identiques ou différents choisis parmi les halogènes et les groupes alkyles en C₁-C₆, alcoxy en C₁-C₆ et nitro,
en présence d'un solvant inerte, avec un alcoolate de formule (III)
M(OR^{³})ₙ (III),
dans laquelle
M représente un métal alcalin ou alcalino-terreux,
R³ représente un groupe alkyle en C₁-C₁₀, cycloalkyle en C₃ -C₁₀ ou aralkyle en C₇-C₁₂ et
n est égal à 1 ou 2 selon la valence de M,
ce qui donne un sel de l'ester cyclopentanone-2-carboxylique de formule (IV) 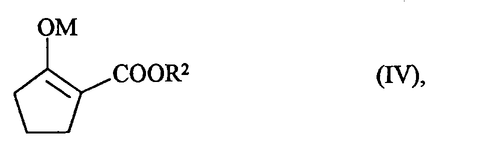 dans laquelle
R² a les significations indiquées en référence à la formule (II) et
M représente un équivalent d'un métal alcalin ou alcalino-terreux,
qu'on alkyle, sans l'isoler, à l'aide d'un agent alkylant de formule (V)
R¹-X (V),
dans laquelle
R¹ a les significations indiquées en référence à la formule (I) et
X représente un halogène, un groupe alkylsulfonate en C₁-C₆ ou un groupe arylsulfonate en C₆-C₁₀ portant éventuellement jusqu'à deux substituants alkyles en C₁-C₄,
ce qui donne un ester 2-alkylcyclopentanone-2-carboxylique de formule (VI) 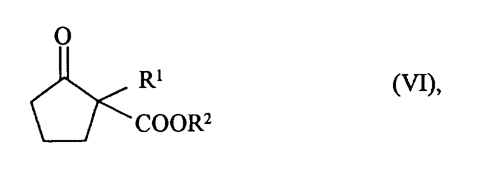 dans laquelle
R² a les significations indiquées en référence à la formule (II) et
R¹ a les significations indiquées en référence à la formule (I),
qu'on soumet sans l'isoler à saponification et décarboxylation par traitement à l'aide d'un acide à chaud.

2. Procédé selon la revendication 1, caractérisé en ce que, dans la formule (II), les deux symboles R² ont la même signification et représentent des groupes alkyles à chaîne droite ou ramifiée en C₁-C₆, des groupes benzyles portant éventuellement jusqu'à deux substituants choisis parmi les atomes d'halogènes et les groupes alkyles en C₁-C₄, ou des groupes phényles portant éventuellement jusqu'à deux substituants choisis parmi les groupes alkyles en C₁-C₄ et les groupes nitro, dans la formule (III), M représente le sodium, le potassium, le lithium, le calcium ou le magnésium et R³ un groupe alkyle à chaîne droite ou ramifiée en C₁-C₆, cycloalkyle en C₃-C₇ ou benzyle, les solvants utilisés sont des hydrocarbures aliphatiques ou aromatiques ayant des points d'ébullition d'au moins 80°C à pression normale ou des hydrocarbures aromatiques substitués par des halogènes et/ou des groupes alcoxy en C₁-C₄, ou des mélanges de ces solvants, et l'alcoolate de formule (III) est mis en oeuvre à l'état de solution dans l'alcool correspondant ; et l'alcool est distillé avant que la réaction avec l'ester adipique de formule (II) se soit déroulée dans une mesure notable.

3. Procédé selon les revendications 1 et 2, caractérisé en ce que l'on ajoute au solvant des solvants aprotoniques dipolaires.

4. Procédé selon les revendications 1 à 3, caractérisé en ce que, pour 1 mol de l'ester adipique de formule (II), on utilise de 0,1 à 10 équivalents d'un alcoolate de formule (III) et on effectue la réaction à des températures dans l'intervalle de 80 à 200°C.

5. Procédé selon les revendications 1 à 4, caractérisé en ce que l'on utilise un agent alkylant de formule (V) dans laquelle R¹ représente un groupe méthyle, éthyle, n-propyle, isopropyle, benzyle, 4-chlorobenzyle ou 4-fluorobenzyle et X représente le chlore, le brome ou un groupe p-tolylsulfonate.

6. Procédé selon les revendications 1 à 5, caractérisé en ce que, pour 1 mol de l'ester adipique de formule (II), on utilise 1 à 20 mol d'un agent alkylant de formule (V), on procède à l'alkylation à des températures de 0 à 250°C et après l'alkylation, on élimine le solvant et le cas échéant l'excès d'agent alkylant.

7. Procédé selon les revendications 1 à 6, caractérisé en ce que le traitement à l'aide d'un acide est réalisé à l'aide de l'acide bromhydrique, de l'acide sulfurique, d'acides alkylsulfoniques ou d'acides arylsulfoniques, ces acides étant mis en oeuvre à l'état de solutions aqueuses.

8. Procédé selon les revendications 1 à 7, caractérisé en ce que, pour 1 mol de l'ester adipique de formule (II), on utilise 1 à 10 équivalents de l'acide.

9. Procédé selon les revendications 1 à 8, caractérisé en ce que la 2-cyclopentanone substituée en position 2 et répondant à la formule (I) est isolée après sa préparation par distillation azéotropique.
